# EUROPEAN PATENT SPECIFICATION

(11) **EP 1 413 255 B1**
(45) Date of publication and mention of the grant of the patent: **09.12.2009**
(21) Application number: 02023955.4
(22) Date of filing: 25.10.2002
(51) Int. Cl.: A61B 17/00

(54) **Absorbable surgical sealing device**
Resorbierbare, medizinische Vorrichtung zur chirurgischen Abdichtung
Dispositif résorbable de fermeture des perforations chirurgicales

(43) Date of publication of application: 28.04.2004
(73) Proprietor: RADI MEDICAL SYSTEMS AB, 754 50 Uppsala (SE)
(72) Inventor: Egnelöv, Por, 75440 Uppsala (SE)
(74) Representative: Lindgren, Anders

(56) References cited:
- EP-A- 1 169 968
- DE-U- 20 009 815
- US-B1- 6 383 201

## Description

### FIELD OF THE INVENTION

The present invention relates to a device for sealing a puncture in a blood vessel according to the preamble of claim 1.

### BACKGROUND OF THE INVENTION

During certain types of medical surgery or treatment, an introducer is used to access the vascular system of a patient. The introducer is inserted through the wall of a blood vessel in order to obtain access to the vascular system, and may thereafter be used for guiding medical instruments such as catheters, guide wires and the like. After completion of the medical procedure, there will be an incision or a wound in the wall of the blood vessel corresponding to the size of the introducer. The bleeding from the wound, which is a result of such a surgical operation, can be stopped by applying direct pressure on the wound. However, applying direct pressure on the wound requires assistance of medical personnel and may also restrict the flow of blood through the vessel.

Through US 5,350,399 is disclosed a sealing device for sealing a puncture in the wall of a blood vessel. This sealing device comprises an intra-arterial occluder and an extra-arterial occluder, which, in a sealing position, are held together by a guide means being integral with and extending centrally from the intra-arterial occluder. According to US 5,350,399, the guide means, which can be in the form of an elongated flexible wire, as well as the occluders can be made from a bioabsorbable material. Further, each occluder is formed of a material and has a shape so as to be circumferentially collapsible from a normal position, and should be resiliently expandable from the collapsed state to the normal position. Such absorbable (e.g. biodegradable) materials that also are resilient materials are usually known to have low rupture strength and the risk that the fastening means, in this case in the form of a guide means, will rupture through the intra-arterial occluder is present.

EP 1 169 968 B1 is the closest prior art and discloses the features of the preamble of claim 1. EP 1 169 968 B1, discloses a sealing device for closing a wound, whose aim is to enhanced tightening properties. The sealing device according to EP 1 169 968 B1 comprises an occlusion member to be fitted against the inner wall of a vessel, a locking member to be fitted against the outer vessel wall, and a retaining element, such as a filament means, connecting the occlusion member and the locking member, so that, in use, force is needed to displace the retaining element once stitched through an implanted object, which brings the occlusion member in tension. There is a potential risk that the tension in the filament means will cause the filament means to rupture through the occlusion member, thereby leaving the occlusion member loose inside the vessel.

In this context, it should be noted that the problem that an inner seal, i.e. a sealing member designed to be positioned against the inner wall of a blood vessel, will come loose in the artery has severe implications both on long and short terms. If the retaining means ruptures through the inner seal during the introduction or shortly after its introduction, i.e. before haemostasis is obtained, the immediate problem is, of course, to stop the flow of blood through the puncture wound. For this incident, when a sealing operation is carried out using this type of intra-arterial occluder, a device for applying external compression pressure on the puncture site is often kept prepared as a precaution. If, however, the retaining means ruptures through the inner seal when haemostasis already is obtained, the problem is that the inner seal can follow the flow of blood to a position where the artery is so narrow that the inner seal occludes the blood vessel, which may necessitate amputation of the part of the body in which the inner seal has got stuck. Having in mind that it normally takes several months before the body actually absorbs arterial sealing devices being made of an absorbable material, it is easy to realize that the long-term requirements regarding the rupture strength of such sealing devices are quite severe.

It should also be noted that a requirement for an intra-arterial sealing device is that it is resilient, since it usually has to be folded, collapsed or in some other way deformed in order to fit in some kind of introducer means before the introduction through the puncture hole and into the vessel. When positioned inside the vessel, the sealing device is unfolded or expanded so as to seal the puncture in the vessel wall. In other words, the diameter of the sealing device must be smaller than the diameter of the puncture hole in the introduction phase, whereas the diameter of the sealing device must be larger than the diameter of the puncture hole in the sealing phase. Generally speaking, the problem is that absorbable (e.g. biodegradable) materials having these properties, i.e. being characterized by having a low modulus, usually also are characterized by having low rupture strength. The rupture strength referred to herein relates to the force needed to displace an implanted object, which is fixed by some fastening or retaining means, such as sutures, filaments, screws or other fasteners or retainers used to fix the object in position relative to the surrounding soft or hard tissue, or the force needed to displace the fastening or retaining means once stitched through the implanted object. The rupture strength of a material is related to the modulus (commonly also referred to as the elastic modulus or Young's modulus) of the material, so that a low modulus material is characterized by having low rupture strength. A high modulus material has a higher resistance to force.

### SUMMARY OF THE INVENTION

The objective problem of the present invention is therefore to provide an improved arterial sealing device that provides safer sealing of a percutaneous puncture.

The problem is solved by a device having the features of claim 1.

Thanks to that the retaining assembly in the sealing device has at least two loops which engage the inner seal such that force exerted by the retaining assembly on the inner seal is distributed over the inner seal, the risk for the retaining assembly to rupture the inner seal is reduced which accordingly gives a safer sealing of a percutaneous puncture.

In one embodiment of the invention, the invention provides a device for sealing a puncture in a blood vessel comprising an inner seal configured to be placed inside a blood vessel and to seal a puncture in a blood vessel by contacting an inner surface of the blood vessel essentially without deforming the blood vessel wall.

In a further embodiment, the inner seal has such a shape that it adapts to the surface of the inner vessel wall, so that an efficient sealing is achieved with a minimum of tension being applied to the retaining member, thereby further reducing the risk that the retaining member ruptures through the inner seal.

In a further embodiment, a locking member has such a shape that it can act as an outer seal in the event that the inner seal should not seal the puncture properly.

### BRIEF DESCRIPTION OF THE DRAWINGS

- Fig. 1: is a cross-sectional view of a wound closure device positioned around a puncture hole in a vessel according to prior art.
- Fig. 2: shows a cross-section of the retaining means and the intra-arterial sealing member of Fig. 1.
- Fig. 3: is a cross-sectional view of a sealing device implanted in a sealing position around a puncture in the wall of a vessel according to the present invention.
- Fig. 4: shows a cross-section of an inner seal according to the present invention.
- Fig. 5: shows a cross-section of a retaining member and the inner seal of Fig. 4.
- Fig. 6: illustrates an alternative embodiment in which two retaining members are connected to an inner seal.
- Fig. 7: illustrates the threading of the retaining members of Fig. 6.
- Fig. 8: is a schematic view of the inner seal of Fig. 3.
- Fig. 9: is a cross-sectional end view of one embodiment of the inner seal according to the present invention.
- Fig. 10: is a schematic view of the under side of the inner seal of Fig. 9.
- Fig. 11: is a schematic view of the upper side of the inner seal of Fig. 9.
- Fig. 12: shows a cross-section of a locking member according to the present invention.

### DETAILED DESCRIPTION OF THE PREFERRED EMBODIMENTS

In order to effectively seal an arterial puncture wound, an absorbable sealing device for intra-arterial (or extra-arterial) sealing should preferably be made of a material that is characterized by being soft and flexible, i.e. the material is characterized by having a low modulus. As discussed above, it is also important that the device be deformable to fit in the introducer and also be capable of unfolding or expanding in the blood vessel. Such materials can be made from natural biopolymers or from synthetic materials that degrade into harmless components within a living tissue. Examples of materials may be various natural biopolymers, pure or chemically manipulated, based on alginic acid, hyalauronic acid or chitosan. Examples of soft and flexible synthetic absorbable polymers are aliphatic polyurethanes, polyphospazenes and polyorthoesters, and those polymers made from glycolide, lactide, caprolactone, trimethylene carbonate, butyrolactone, 1,4-dioxan-2-one or 1,5-dioxepan-2-one. Yet another way to achieve a soft and flexible material is the use of plasticizers to bring down the glass transition temperature of the material in question. Such suitable materials have an elastic modulus that ranges from 50 to 120 MPa, and more preferably from 60 to 80 MPa.

As described above, soft and flexible absorbable materials normally have a low rupture strength, which, in turn, means that there is a potential risk that a retaining means, such as a multifilament or a suture, is torn lose by rupturing through a sealing member being made from such materials. During the implantation procedure, the doctor needs to feel that the sealing member is pulled in close apposition to the vessel wall. During this procedure, the doctor needs to pull with some force, which is applied with great individual variability and which will be transmitted through the retaining means and act on the sealing member. With the conventional way of threading the retaining member, e.g. through two through holes in an intra-arterial sealing member, there is therefore a potential risk that the retaining member saws its way through the material in the sealing member between the two through holes, thereby leaving the sealing member loose inside the vessel. In order to overcome this potentially severe problem, a new way of threading the retaining member is proposed, as set forth below.

Further, when correctly positioned against the inner wall of a vessel, the intra-arterial sealing member according to the present invention has the ability to seal a puncture hole with a minimum of tension being applied in the retaining member. This is an advantage since the smaller the tension in the retaining member, the less the risk that the retaining means ruptures through the intra-arterial sealing member.

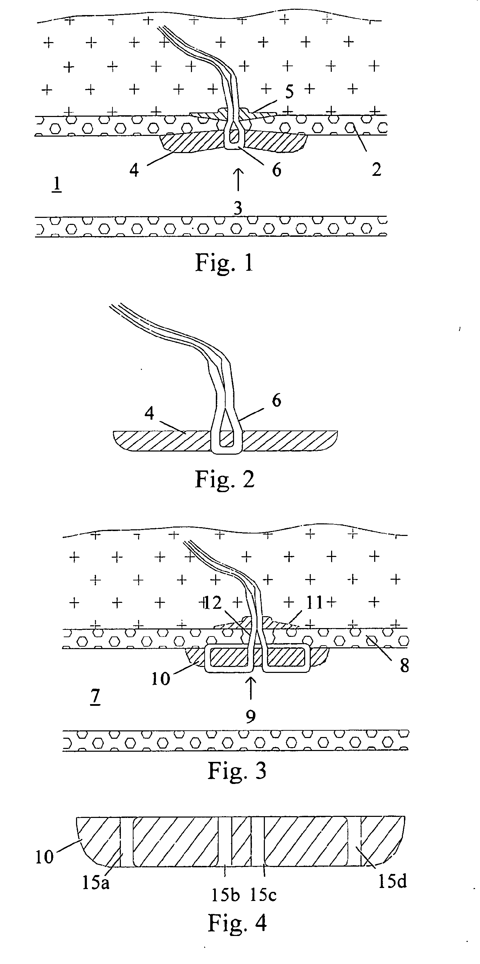

In Fig. 1 is illustrated a portion of a vessel 1 in a living body, such as the femoral artery. A puncture has been made through the vessel wall 2, thereby creating an opening, which has to be occluded after the treatment that made the puncture necessary. In Fig. 1, a previously proposed wound closure device 3 has been implanted to close the puncture wound. The wound closure device 3 comprises an intra-arterial sealing member 4, which is positioned against the inner surface of the vessel wall 2, and an extra-arterial sealing member 5, which is positioned against the outer surface of the vessel wall 2. The wound closure device 3 comprises also a fastening or retaining means 6 in the form of a multifilament 6, which holds the intra-arterial and extra-arterial sealing members 4, 5 together by means of friction locking. As is usual in this type of device, this wound closure device 3 seals the puncture in the vessel 1 by clamping the vessel wall 2 between the intra-arterial sealing member 4 and the extra-arterial sealing member 5.

As is best seen in Fig. 2, the intra-arterial sealing member 4 comprises also two through holes, through which the multifilament 6 is threaded in a single loop. With this device, there is therefore a relatively small amount of material between the two through holes, which, as mentioned above, implies that there is a risk that the multifilament 6 ruptures through the intra-arterial sealing member 4 because the multifilament 6 concentrates force on member 4 over a small area.

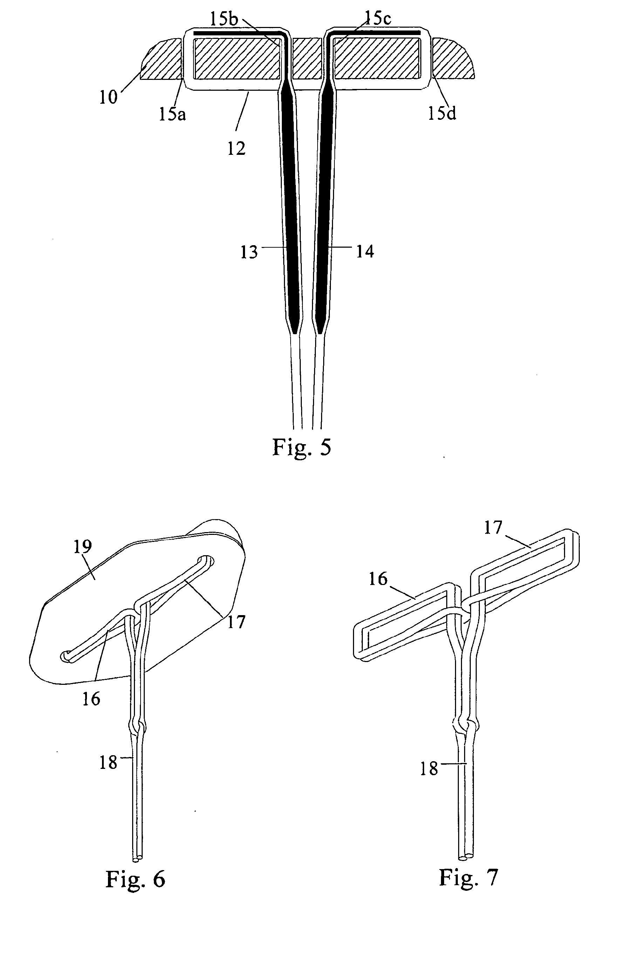

Fig. 3 illustrates the same type of puncture through the wall 8 of a vessel 7. In this case, a sealing device 9 according to the present invention has been positioned to close the puncture wound in the vessel wall 8. The sealing device 9 comprises basically three separate parts, namely an inner seal 10, which is adapted to be positioned against the inner surface of the vessel wall 8, a locking member 11, which is to be positioned against the outer surface of the vessel wall 8, and a retaining assembly in the form of a retaining member 12. These components are made from bioabsorbable materials. In this embodiment, the retaining member 12 is in the form of a multifilament 12, which extends through the puncture hole and connects the inner seal 10 with the locking member 11. The retaining member can be any other type of thread such as a suture. As is seen in Fig. 5, the retaining member 12 comprises also two elongated members 13, 14, which are inserted in the distal portion of the multifilament 12. The function of these elongated members 13, 14 is to provide a secure locking for the locking member 11 by means of friction. During implantation of the sealing device 9, when the inner seal 10 has been pulled in close apposition to the inner surface of the vessel wall 8, the locking member 11 can easily slide along the thin proximal portion of the multifilament 12 until it is pushed up on and along the thicker distal portion of the multifilament 12 and into contact with the outer surface of the vessel wall 8, where it remains securely seated by the friction locking in order to hold the inner seal 10 in place.

Instead of inserting elongated members into a multifilament, it is also possible to provide the extra thickness at the distal end of a multifilament, and thereby the friction locking for the locking member, by other means. A multifilament could, for example, be coated or dressed with some extra material, so that the extra thickness is provided from the outside rather than from the inside of the multifilament. An alternative locking arrangement is shown in Fig. 6 and Fig. 7, wherein, at the distal end, the multifilament is doubled up to provide a friction lock.

As can be seen from Fig. 3, the vessel wall 8 is much less deformed than the vessel wall 2 of Fig. 1. This feature reflects the fact that the sealing device 9 preferably seals the puncture hole by holding the inner seal 10 in intimate engagement with the inner surface of the vessel wall 8, or, in other words, there is only a minimal amount of tension applied in the filament 12. This way of sealing a puncture hole is in contrast to the prior art sealing devices, wherein a puncture hole is sealed by clamping the vessel wall between an intra-arterial sealing member and an extra-arterial sealing member. The present way of sealing the puncture hole is advantageous in that less tension has to be applied in the multifilament 12, which, in turn, means that there is less risk that the multifilament 12 ruptures through the inner seal 10. This way of sealing a puncture hole requires that the inner seal 10 to a large extent has the ability to adapt to the structure of the inner surface of the vessel wall 8. The characteristics of the inner seal 10 will be described in more detail below.

The cross-section of the inner seal 10 is shown in Fig. 4, where it can be seen that the inner seal 10 also comprises in total four through holes, two outer holes 15a and 15d and two inner holes 15b and 15c. As already is indicated in Fig. 3 and more fully described in conjunction with Fig. 5 below, the multifilament 12 is threaded through these four through holes 15a-d in such a way that the strain on the inner seal 10 is reduced in comparison with the conventional way of threading a retaining member through, for example, only one or two through holes.

The way in which the multifilament 12 is threaded through the four through holes 15a-d is illustrated in Fig. 5. As can be seen from the figure, the multifilament 12 is threaded such that the ends of the multifilament 12 are threaded through the two outer holes 15a and 15d, from the under side of the inner seal 10 to its upper side, and the ends of the multifilament 12 are then threaded through the two inner holes 15b and 15c, from the upper side of the inner seal 10 to its under side, so that two separate loops are formed. Herein, the under side of the inner seal 10 is defined as the side of the inner seal 10 that is to be positioned in engagement with the vessel wall, whereas the upper side of the inner seal 10 is the side that will face the interior of the vessel. A perhaps simpler way to describe the threading of the multifilament 12 is to note that the threaded multifilament 12 is characterized by having two separate portions on the upper side of the inner seal 10, while there is only one portion on the under side of the inner seal 10.

It could also be mentioned that it is possible to let the two inner holes 15b and 15c be merged into a single larger hole, which is positioned in the centre of the inner seal and which receives both ends of the multifilament. In principle, it is also possible to provide an inner seal with more than four holes, so that a retaining member is threaded in more than two loops. Yet another way to achieve the same effect, i.e. to reduce the strain on the inner seal, would be to use more than one retaining member. An example of the latter is illustrated in Fig. 6 and Fig. 7, where two retaining members 16, 17 have been threaded through an inner seal 19 such that two separate loops are formed. Outside of the inner seal 19, the two retaining members 16, 17 are connected by a third retaining member 18, so that a configuration having the same function as the combination of the multifilament 12 and the elongated members 13, 14 shown in Fig. 5 is formed. As already has been mentioned, there are different ways of providing the friction locking, i.e. of arranging the retaining assembly. However, a common requirement is that a central part of the retaining assembly, i.e. the part that extends through the puncture hole, occupies a diameter that is less than or equal to the diameter of the puncture hole, so that the edges of the puncture hole are not damaged by the retaining member(s).
The advantage with the new threading of the retaining member is that the outer through holes are positioned such that the distance between them are larger than the diameter of the puncture hole, thereby allowing the retaining member to enclose more material so that the risk that the retaining member ruptures through the inner seal is significantly reduced in comparison with the sealing device shown in Fig. 1.

From a comparison of the intra-arterial sealing member 4 illustrated in Fig. 2 and the inner seal 10 illustrated in Fig. 5 it should now be possible to fully appreciate a special advantage of the present invention. With the present design of the inner seal 10 and the improved threading of the retaining member 12, the force from the retaining element 12 that acts on the inner seal 10 is spread out over an area that is much larger than the corresponding area of the sealing member 4. Consequently, the risk that the retaining element 12 ruptures through the inner seal 10 has been significantly reduced. Furthermore, the two loops of the retaining member 12 enclose more material than does the single loop, which means that the retaining element 12 has to saw its way through more material.

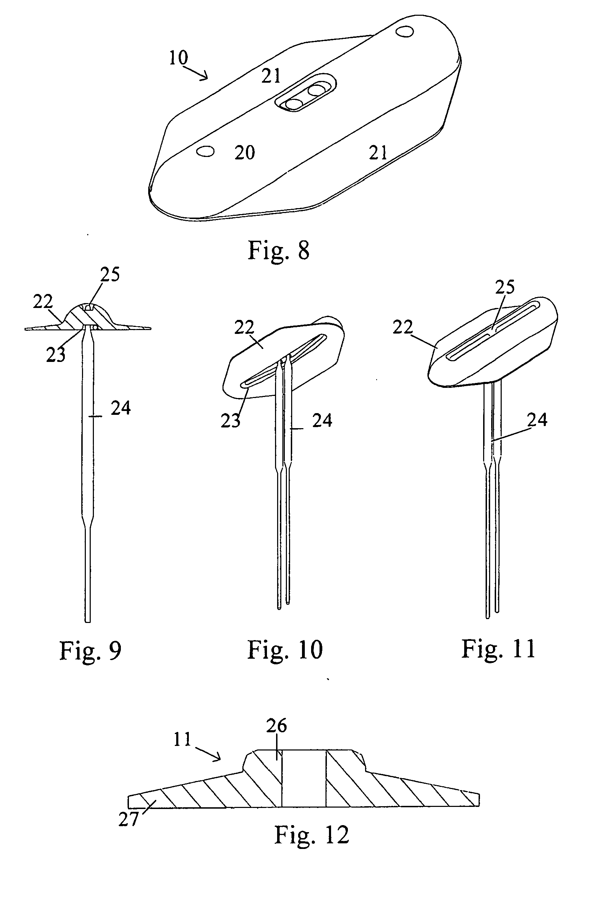

As mentioned above, to seal a puncture hole with only a minimum of tension being applied in a retaining member puts special requirements on an inner seal, i.e. it has to be resilient and soft enough to adapt to the surface of the vessel wall. Fig. 8 shows the inner seal 10 according to the present invention. As can be seen from the figure, the inner seal 10 includes a central, elongated portion 20 surrounded by a rim portion 21. The central portion 20, together with the improved way of threading the retaining member, provides the inner seal 10 with the necessary stiffness and rupture strength, while the comparatively thinner rim portion 21 provides the resilience which is necessary for the inner seal to adapt to the surface of the vessel wall so as to seal a puncture hole without actually clamping the vessel wall between the inner seal 10 and the locking member 11.

Before further describing the sealing device according to the present invention, a few words could be said about the tension in the retaining member and the way that the inner seal seals a puncture hole in the wall of a vessel. The tension in a retainer, which extends from an inner member positioned against the inner vessel wall, through a puncture hole in the vessel wall and to an outer member positioned against the outer vessel wall, can range continuously from zero to a large value. With a resilient inner member, which adapts perfectly to the surface of the inner vessel wall, it is, at least theoretically, possible to seal the puncture hole without applying any tension in the retainer, i.e. when the length of the retainer between the inner member and the outer member matches exactly the thickness of the vessel wall. The other extreme, i.e. a large tension in the retainer, corresponds to the case when the puncture hole is sealed by clamping the vessel wall between the inner member and the outer member. In the latter case, it is neither necessary nor important that the inner member is resilient, since the sealing is achieved by deformation of the vessel wall, i.e. the vessel wall adapts to the inner member in such a way that the fluid inside the vessel cannot penetrate into the puncture hole.

From the above, it should be clear that the sealing device according to the present invention preferably belongs to a class of sealing devices comprising an inner seal, which seals a puncture by adapting to the inner surface of the vessel wall, and a locking member, which is positioned at the outer vessel wall and whose primary function is to hold the inner seal in place. With such a sealing device, the tension in a retaining member can be reduced to a minimum, which is in contrast to sealing devices that seal a puncture wound by clamping and deforming the vessel wall between an inner member and an outer member. A minimal tension could therefore be defined as the amount of the tension required to make the inner seal to adapt to the inner surface of the vessel wall. For example, a small amount of tension in the retaining member is required to unfold the thin rim portion of the inner seal according to the present invention, since the inner seal, before the introduction, has been compressed inside an introducer. Further, if only a minimal tension is going to be applied, the friction locking of the locking member has to be continuously variable, i.e. the locking member has to be continuously movable along the distal portion of the retaining member. This is, for example, in contrast to devices in which an outer member is secured by saw teeth provided on a retaining member. With the present invention, the tension in the retaining member, immediately after completion of the positioning of the inner seal and the locking member, is approximately less than 1 N and approaches zero when the sealing device has been in place for a while, about three weeks. Here, one has to remember that, as is usual within the art, the sealing device according to the present invention preferably is made from an absorbable material that slowly degrades inside the body. This means that the sealing device, during its lifetime, will be in different states of dissolution or degradation, and the risk that the retaining member ruptures through the inner seal would, without the improved threading of the retaining member, still be present even though the tension in the retaining member is very small. In other words, the improved way of threading the retaining member and the small tension in the retaining member, which is due in part to the resilient rim portion of the inner seal, are complementary, which is not to say that they can not perform their functions independently of each other.

For the ability of the inner seal to adapt to the surface structure of the vessel wall, it may be an advantage that the under side of the inner seal is flat, i.e. that there are no elevated parts. Apparently, this is not the case for the combination of an inner seal and retaining member shown in Fig. 3 and Fig. 5, where the thickness of the multifilament 12 has been increased for the sake of clarity. Fig. 9 shows an end view of an alternative embodiment of an inner seal 22 according to the present invention. As can be seen from Fig. 9 and also from Fig. 10, the under side of the inner seal 22 is provided with a longitudinal recess 23, in which a loop of a retaining member 24 is placed so that the periphery of the retaining member 24 is in level with the under side of the inner seal 22. This feature facilitates the ability of the inner seal 22 to adapt to the surface of a vessel wall, since there are no parts projecting out from the under side of the inner seal 22.

As can be seen from Fig. 9 and also from Fig. 11, the upper side of the inner seal 22 is also provided with a longitudinal recess 25, in which the two loops (as was described in conjunction with Fig. 5) of the retaining member 24 are placed so that the periphery of the retaining member 24 is in level with the upper side of the inner seal 22. With this positioning of the retaining member 24, the inner seal 22 exhibits a flat upper side, which obstructs the fluid flow in a vessel less than an upper side provided with a projecting retaining member.

The provision of one or both of the recesses 23, 25 is optional, and the importance of embedding the retaining member in, for example, a recess in the under side of the inner seal depends on the specific design and particular choice of material for the inner seal in question. Thus, if the material from which the inner seal is made is soft and resilient enough, it may not be necessary to provide the above-mentioned recesses, but for other choices of material and other designs it might be advantageous.

In Fig. 12 is shown a cross-section of the locking member 11 shown in Fig. 3. From the above-mentioned fact that the primary function of the locking member 11 is to hold the inner seal 10 seated against the inner surface of a vessel wall, it follows that the locking member 11 in principle could have any shape, as long as the requirements arising from the dimensions of an introducer, the frictional locking between the locking member 11 and the retaining member 12, and the size of the puncture hole are fulfilled. However, if, for some reason, the inner seal 10 does not seal the puncture properly, so that some fluid from the interior of the vessel penetrates into the puncture hole, the locking member 11 should preferably have such a shape that it can act as an outer seal 11 and, if necessary, assist in the sealing of the puncture. For that incident, the locking member 11 has basically the same structure as the inner seal 10, with a thick central portion 26 surrounded by a thin rim portion 27. The thicker central portion 26 provides the necessary mechanical strength for a secure friction locking between the locking member 11 and the retaining member 12, while the thinner rim portion 27 provides the resilience which is necessary for the ability of the locking member 11 to adapt to the outer surface of the vessel wall and thereby, if necessary, seal the puncture from the outside of the vessel wall.

The improved way of threading can also be used in a situation where there exists a large tension in the retaining assembly, even if the vessel wall is deformed by this tension, because the improved way of threading minimizes the risk that the retaining assembly will rupture or saw through the inner seal.

Although the present invention has been described with reference to specific embodiments, also shown in the appended drawings, it will be apparent for those skilled in the art that many variations and modifications can be done within the scope of the invention as described in the specification and defined with reference to the following claims.

## Claims

1. A device (9) for sealing a puncture in a blood vessel, comprising an inner seal (10) configured to be placed inside a blood vessel and to seal a puncture in the blood vessel by contacting an inner surface wall of the blood vessel, the device (9) comprising a retaining assembly (12), wherein the inner seal (10) comprises a thick central elongated portion (20) at least partially surrounded by a thin rim portion (21);
**characterized in that**
the retaining assembly (12) has at least two loops which engage the inner seal (10); and **in that** the loops extend in the longitudinal direction of the thick central elongated portion (20), whereby force exerted by the retaining assembly
(12) on the inner seal (10) is distributed over the inner seal (10).

2. The device (9) according to claim 1, wherein the retaining assembly (12) is configured to retain the inner seal (10) in contact with the inner surface wall of the blood vessel with a minimal tension being applied in the retaining assembly (12).

3. The device (9) according to any of the claims 1-2, wherein the retaining assembly (12) is configured to retain the inner seal (10) in contact with the inner surface wall of the blood vessel such that the tension in the retaining assembly (12) is less than 1 N.

4. The device (9) according to any of the claims 1-3, further comprising a locking member (11) configured to slide along the retaining assembly (12) and contact an outer surface wall of the blood vessel.

5. The device (9) according to any of the claims 1-4, wherein the locking member (11) is configured to seal the puncture from outside the blood vessel.

6. The device (9) according to any of the claims 1-5, wherein the retaining assembly (12) comprises a thread.

7. The device (9) according to any of the claims 1-6, wherein the inner seal (10) comprises at least three holes (15a-d) through which the retaining assembly (12) extends.

8. The device (9) according to claim 4-7, wherein the retaining assembly (12) comprises a multifilament and at least one elongated member (13;14) inserted in a distal portion of the multifilament to make the multifilament thicker.

9. The device (9) according to any of the claims 6-8, wherein each end of the thread is threaded from an under side of the inner seal (10) to an upper side of the inner seal (10) via two outermost holes (15a,d) of the at least three holes (15a-d) and then from the upper side to the under side via at least one innermost hole (15b,c) of the at least three holes (15a-d) to form the at least two loops.

10. The device (9) according to any of the claims 6-9, wherein the thread is threaded through the inner seal such that the thread has two separate portions on an upper side of the inner seal and one integral portion on an under side of the inner seal.

11. The device (9) according to any of the claims 1-10, wherein the retaining assembly (12) comprises one retaining member which forms both loops.

12. The device (9) according to any of the claims 1-10, wherein the retaining assembly (12) comprises a first retaining member which forms one of the two loops and a second retaining member which forms the other of the two loops.

13. The device (9) according to any of the claims 1-12, wherein the inner seal (10) comprises a recess (23) in an under side of the inner seal (10) to house at least a portion of the retaining assembly (12) such that a periphery of the retaining assembly (12) does not project from the under side.

14. The device (9) according to any of the claims 1-13, wherein the inner seal (10) comprises a recess (25) in an upper side of the inner seal (10) to house at least a portion of the retaining assembly (12) such that a periphery of the retaining assembly (12) does not project from the upper side.

15. The device (9) according to any of the claims 4-14, wherein the locking member (11) comprises a thick central portion (26) at least partially surrounded by a thin rim portion (27).

16. The device (9) according to any of the claims 5-15, wherein the retaining assembly (12) comprises a thread which has been doubled up at least one time to make the thread thicker.

17. The device (9) according to any of the claims 1-16, wherein the inner seal (10) is configured to contact the inner surface wall of the blood vessel around the entire circumference of the puncture.

18. The device (9) according to any of the claims 1-17, wherein the retaining assembly (12) is made of a bioabsorbable material.

19. The device (9) according to any of the claims 1-18, wherein the inner seal (10) is made of a bioabsorbable material.

20. The device (9) according to any of the claims 4-19, wherein the locking member (11) is made of a bioabsorbable material.

21. The device according to any of the claims 18-20, wherein the bioabsorbable material has an elastic modulus that ranges from 50 to 120 MPa.

22. The device according to any of the claims 18-20, wherein the bioabsorbable material has an elastic modulus that ranges from 60 to 80 MPa.

23. The device (9) according to any of the claims 1-20, wherein the retaining assembly (12) is configured to retain the inner seal (10) in contact with the inner surface wall of the blood vessel essentially without deforming the vessel wall.

24. The device (9) according to any of the claims 1-23, wherein the retaining assembly (12) comprises a thread whose distal end has been coated or dressed to make the distal end thicker.

25. The device (9) according to any of the claims 1-24, further comprising a third retaining member to connect the first retaining member and the second retaining member.

26. The device (9) according to any of the claims 1-23, wherein a diameter of the retaining assembly (12) is increased at a distal end to friction lock the locking member (11) and the retaining assembly (12).

## Patentansprüche

1. Vorrichtung (9) zum Verschließen einer Punktur in einem Blutgefäß, mit einem inneren Verschluss (10), der zum Platzieren innerhalb eines Blutgefäßes und zum Verschließen einer Punktur in dem Blutgefäß durch Berühren einer Innenflächenwand des Blutgefäßes konfiguriert ist, wobei die Vorrichtung (9) eine Halteanordnung (12) umfasst, wobei der innere Verschluss (10) einen dicken, mittleren, länglichen Teil (20) umfasst, der zumindest teilweise von einem dünnen Randteil (21) umgeben ist;
**dadurch gekennzeichnet, dass**
die Halteanordnung (12) mindestens zwei Schlaufen aufweist, die den inneren Verschluss (10) in Eingriff nehmen, und dass sich die Schlaufen in Längsrichtung in dem dicken, mittleren, länglichen Teil (20) erstrecken, wobei durch die Halteanordnung (12) auf den inneren Verschluss (10) ausgeübte Kraft über den inneren Verschluss (10) verteilt wird.

2. Vorrichtung (9) nach Anspruch 1, wobei die Halteanordnung (12) dazu konfiguriert ist, den inneren Verschluss (10) mit der Innenflächenwand des Blutgefäßes in Kontakt zu halten, wobei minimale Spannung in der Halteanordnung (12) angelegt wird.

3. Vorrichtung (9) nach einem der Ansprüche 1 - 2, wobei die Halteanordnung (12) dazu konfiguriert ist, den inneren Verschluss (10) mit der Innenflächenwand des Blutgefäßes so in Kontakt zu halten, dass die Spannung in der Halteanordnung (12) weniger als 1 N beträgt.

4. Vorrichtung (9) nach einem der Ansprüche 1 - 3, die weiterhin ein Verriegelungsglied (11) umfasst, das dazu konfiguriert ist, entlang der Halteanordnung (12) zu gleiten und eine Außenflächenwand des Blutgefäßes zu berühren.

5. Vorrichtung (9) nach einem der Ansprüche 1 - 4, wobei das Verriegelungsglied (11) dazu konfiguriert ist, die Punktur von außerhalb des Blutgefäßes zu verschließen.

6. Vorrichtung (9) nach einem der Ansprüche 1 - 5, wobei die Halteanordnung (12) einen Faden umfasst.

7. Vorrichtung (9) nach einem der Ansprüche 1 - 6, wobei der innere Verschluss (10) mindestens drei Löcher (15a-d) umfasst, durch die sich die Halteanordnung (12) erstreckt.

8. Vorrichtung (9) nach einem der Ansprüche 4 - 7, wobei die Halteanordnung (12) ein Multifilament und mindestens ein längliches Glied (13; 14) umfasst, das in einem distalen Teil des Multifilaments eingesetzt ist, um das Multifilament dicker zu machen.

9. Vorrichtung (9) nach einem der Ansprüche 6 - 8, wobei jedes Ende des Fadens von einer Unterseite des inneren Verschlusses (10) über zwei äußerste Löcher (15a,d) der mindestens drei Löcher (15a-d) zu einer Oberseite des inneren Verschlusses (10) und dann von der Oberseite über mindestens ein innerstes Loch (15b, c) der mindestens drei Löcher (15a-d) zur Unterseite gefädelt wird, um die mindestens zwei Schlaufen zu bilden.

10. Vorrichtung (9) nach einem der Ansprüche 6 - 9, wobei der Faden so durch den inneren Verschluss gefädelt wird, dass der Faden zwei getrennte Teile auf einer Oberseite des inneren Verschlusses und einen integralen Teil auf einer Unterseite des inneren Verschlusses aufweist.

11. Vorrichtung (9) nach einem der Ansprüche 1 - 10, wobei die Halteanordnung (12) ein Halteglied umfasst, das beide Schlaufen bildet.

12. Vorrichtung (9) nach einem der Ansprüche 1 - 10, wobei die Halteanordnung (12) ein erstes Halteglied, das eine der beiden Schlaufen bildet, und ein zweites Halteglied, das die andere der beiden Schlaufen bildet, umfasst.

13. Vorrichtung (9) nach einem der Ansprüche 1 - 12, wobei der innere Verschluss (10) eine Aussparung (23) in einer Unterseite des inneren Verschlusses (10) umfasst, um mindestens einen Teil der Halteanordnung (12) aufzunehmen, so dass ein Umfang der Halteanordnung (12) nicht von der Unterseite vorragt.

14. Vorrichtung (9) nach einem der Ansprüche 1 - 13, wobei der innere Verschluss (10) eine Aussparung (25) in einer Oberseite des inneren Verschlusses (10) umfasst, um mindestens einen Teil der Halteanordnung (12) aufzunehmen, so dass ein Umfang der Halteanordnung (12) nicht von der Oberseite vorragt.

15. Vorrichtung (9) nach einem der Ansprüche 4 - 14, wobei das Verriegelungsglied (11) einen dicken, mittleren Teil (26) umfasst, der zumindest teilweise von einem dünnen Randteil (27) umgeben wird.

16. Vorrichtung (9) nach einem der Ansprüche 5 - 15, wobei die Halteanordnung (12) einen Faden umfasst, der mindestens einmal umgefaltet worden ist, um den Faden dicker zu machen.

17. Vorrichtung (9) nach einem der Ansprüche 1 - 16, wobei der innere Verschluss (10) so konfiguriert ist, dass er die Innenflächenwand des Blutgefäßes um den gesamten Umfang der Punktur berührt.

18. Vorrichtung (9) nach einem der Ansprüche 1 - 17, wobei die Halteanordnung (12) aus einem bioresorbierbaren Material hergestellt ist.

19. Vorrichtung (9) nach einem der Ansprüche 1 - 18, wobei der innere Verschluss (10) aus einem bioresorbierbaren Material hergestellt ist.

20. Vorrichtung (9) nach einem der Ansprüche 4 - 19, wobei das Verriegelungsglied (11) aus einem bioresorbierbaren Material hergestellt ist.

21. Vorrichtung nach einem der Ansprüche 18 - 20, wobei das bioresorbierbare Material einen Elastizitätsmodul in einem Bereich von 50 bis 120 MPa aufweist.

22. Vorrichtung nach einem der Ansprüche 18 - 20, wobei das bioresorbierbare Material einen Elastizitätsmodul in einem Bereich von 60 bis 80 MPa aufweist.

23. Vorrichtung (9) nach einem der Ansprüche 1 - 20, wobei die Halteanordnung (12) dazu konfiguriert ist, den inneren Verschluss (10) mit der Innenflächenwand des Blutgefäßes in Kontakt zu halten, im Wesentlichen ohne die Gefäßwand zu verformen.

24. Vorrichtung (9) nach einem der Ansprüche 1 - 23, wobei die Halteanordnung (12) einen Faden umfasst, dessen distales Ende beschichtet oder ummantelt worden ist, um das distale Ende dicker zu machen.

25. Vorrichtung (9) nach einem der Ansprüche 1 - 24, die weiterhin ein drittes Halteglied umfasst, um das erste Halteglied und das zweite Halteglied zu verbinden.

26. Vorrichtung (9) nach einem der Ansprüche 1 - 23, wobei ein Durchmesser der Halteanordnung (12) an einem distalen Ende vergrößert wird, um das Verriegelungsglied (11) und die Halteanordnung (12) reibschlüssig zu verbinden.

## Revendications

1. Dispositif (9) pour fermer une perforation dans un vaisseau sanguin, comprenant un joint intérieur (10) configuré de manière à être placé à l'intérieur d'un vaisseau sanguin et à fermer une perforation dans le vaisseau sanguin en venant en contact avec une surface interne d'une paroi du vaisseau sanguin, le dispositif (9) comprenant un ensemble de retenue (12), le joint intérieur (10) comprenant une portion allongée centrale épaisse (20) au moins en partie entourée par une mince portion de bord (21) ;
**caractérisé en ce que**
l'ensemble de retenue (12) a au moins deux boucles qui s'engagent avec le joint intérieur (10) ; et **en ce que** les boucles s'étendent dans la direction longitudinale de la portion allongée centrale épaisse (20), une force exercée par l'ensemble de retenue (12) sur le joint intérieur (10) étant ainsi répartie sur le joint intérieur (10).

2. Dispositif (9) selon la revendication 1, dans lequel l'ensemble de retenue (12) est configuré de manière à retenir le joint intérieur (10) en contact avec la surface interne de la paroi du vaisseau sanguin avec une tension minimale appliquée dans l'ensemble de retenue (12).

3. Dispositif (9) selon l'une quelconque des revendications 1 à 2, dans lequel l'ensemble de retenue (12) est configuré pour retenir le joint intérieur (10) en contact avec la surface interne de la paroi du vaisseau sanguin de telle sorte que la tension dans l'ensemble de retenue (12) soit inférieure à 1 N.

4. Dispositif (9) selon l'une quelconque des revendications 1 à 3, comprenant en outre un organe de verrouillage (11) configuré pour glisser le long de l'ensemble de retenue (12) et pour venir en contact avec une surface externe de la paroi du vaisseau sanguin.

5. Dispositif (9) selon l'une quelconque des revendications 1 à 4, dans lequel l'organe de verrouillage (11) est configuré de manière à fermer la perforation depuis l'extérieur du vaisseau sanguin.

6. Dispositif (9) selon l'une quelconque des revendications 1 à 5, dans lequel l'ensemble de retenue (12) comprend un fil.

7. Dispositif (9) selon l'une quelconque des revendications 1 à 6, dans lequel le joint intérieur (10) comprend au moins trois trous (15ad) à travers lesquels s'étend l'ensemble de retenue (12).

8. Dispositif (9) selon l'une quelconque des revendications 4 à 7, dans lequel l'ensemble de retenue (12) comprend un multifilament et au moins un organe allongé (13 ; 14) inséré dans une portion distale du multifilament pour rendre le multifilament plus épais.

9. Dispositif (9) selon l'une quelconque des revendications 6 à 8, dans lequel chaque extrémité du fil est enfilée depuis un côté inférieur du joint intérieur (10) jusqu'à un côté supérieur du joint intérieur (10) par le biais de deux trous extérieurs (15a,d) des au moins trois trous (15ad) puis depuis le côté supérieur jusqu'au côté inférieur par le biais d'au moins un trou interne (15b,c) des au moins trois trous (15a-d) pour former les au moins deux boucles.

10. Dispositif (9) selon l'une quelconque des revendications 6 à 9, dans lequel le fil est enfilé à travers le joint intérieur de telle sorte que le fil ait deux portions séparées sur un côté supérieur du joint intérieur et une portion intégrale sur un côté inférieur du joint intérieur.

11. Dispositif (9) selon l'une quelconque des revendications 1 à 10, dans lequel l'ensemble de retenue (12) comprend un organe de retenue qui forme les deux boucles.

12. Dispositif (9) selon l'une quelconque des revendications 1 à 10, dans lequel l'ensemble de retenue (12) comprend un premier organe de retenue qui forme l'une des deux boucles et un deuxième organe de retenue qui forme l'autre des deux boucles.

13. Dispositif (9) selon l'une quelconque des revendications 1 à 12, dans lequel le joint intérieur (10) comprend un retrait (23) dans un côté inférieur du joint intérieur (10) pour loger au moins une portion de l'ensemble de retenue (12) de telle sorte qu'une périphérie de l'ensemble de retenue (12) ne fasse pas saillie depuis le côté inférieur.

14. Dispositif (9) selon l'une quelconque des revendications 1 à 13, dans lequel le joint intérieur (10) comprend un retrait (25) dans un côté supérieur du joint intérieur (10) pour loger au moins une portion de l'ensemble de retenue (12) de telle sorte qu'une périphérie de l'ensemble de retenue (12) ne fasse pas saillie depuis le côté supérieur.

15. Dispositif (9) selon l'une quelconque des revendications 4 à 14, dans lequel l'organe de verrouillage (11) comprend une portion centrale épaisse (26) au moins partiellement entourée par une mince portion de bord (27).

16. Dispositif (9) selon l'une quelconque des revendications 5 à 15, dans lequel l'ensemble de retenue (12) comprend un fil qui a été doublé au moins une fois pour rendre le fil plus épais.

17. Dispositif (9) selon l'une quelconque des revendications 1 à 16, dans lequel le joint intérieur (10) est configuré pour venir en contact avec la surface interne de la paroi du vaisseau sanguin autour de toute la circonférence de la perforation.

18. Dispositif (9) selon l'une quelconque des revendications 1 à 17, dans lequel l'ensemble de retenue (12) est fabriqué en un matériau bioabsorbable.

19. Dispositif (9) selon l'une quelconque des revendications 1 à 18, dans lequel le joint intérieur (10) est fabriqué en un matériau bioabsorbable.

20. Dispositif (9) selon l'une quelconque des revendications 4 à 19, dans lequel l'organe de verrouillage (11) est fabriqué en un matériau bioabsorbable.

21. Dispositif selon l'une quelconque des revendications 18 à 20, dans lequel le matériau bioabsorbable a un module d'élasticité qui va de 50 à 120 MPa.

22. Dispositif selon l'une quelconque des revendications 18 à 20, dans lequel le matériau bioabsorbable a un module d'élasticité qui va de 60 à 80 MPa.

23. Dispositif (9) selon l'une quelconque des revendications 1 à 20, dans lequel l'ensemble de retenue (12) est configuré de manière à retenir le joint intérieur (10) en contact avec la surface interne de la paroi du vaisseau sanguin essentiellement sans déformer la paroi du vaisseau.

24. Dispositif (9) selon l'une quelconque des revendications 1 à 23, dans lequel l'ensemble de retenue (12) comprend un fil dont l'extrémité distale a été revêtue ou apprêtée pour rendre l'extrémité distale plus épaisse.

25. Dispositif (9) selon l'une quelconque des revendications 1 à 24, comprenant en outre un troisième organe de retenue pour connecter le premier organe de retenue au deuxième organe de retenue.

26. Dispositif (9) selon l'une quelconque des revendications 1 à 23, dans lequel un diamètre de l'ensemble de retenue (12) est accru à une extrémité distale pour verrouiller par friction l'organe de verrouillage (11) à l'ensemble de retenue (12).
